# EUROPÄISCHE PATENTSCHRIFT

(11) **EP 0 749 773 B1**
(45) Veröffentlichungstag und Bekanntmachung des Hinweises auf die Patenterteilung: **03.03.1999**
(21) Anmeldenummer: 96105997.9
(22) Anmeldetag: 17.04.1996
(51) Int. Cl.: B01D 53/84

(54) **Anordnung zur Aufbereitung kohlendioxidhaltiger Gase**
System for processing carbon dioxide containing gases
Système pour le traitement de gaz contenant du dioxyde de carbone

(30) Priorität: 21.06.1995 DE 19522429
(43) Veröffentlichungstag der Anmeldung: 27.12.1996
(73) Patentinhaber: Lorenz, Thomas, 49134 Wallenhorst-Hollage (DE)
(72) Erfinder: Lorenz, Thomas, 49134 Wallenhorst-Hollage (DE)
(74) Vertreter: Habbel, Hans-Georg

(56) Entgegenhaltungen:
- EP-A- 0 590 477
- DE-A- 3 130 105
- DE-A- 3 607 864

## Beschreibung

Die Erfindung betrifft eine Anordnung nach dem Oberbegriff des Anspruchs 1.

Eine derartige Anordnung ist aus der DE-A-31 30 105 bekannt. Aus der Zusammenfassung geht der vorzugsweise in südlichen Ländern vorgesehene Einsatz dieser Anordnung hervor. Außer einer günstigen Intensität der Sonneneinstrahlung herrschen dort auch Temperaturen, die ein Algenwachstum begünstigen. Klimaschwankungen und insbesondere tiefe Temperaturen können durch ungünstige Lebensbedingungen für die Algen den Wirkungsgrad der gesamten Anlage erheblich beeinträchtigen.

Aus der DE-A-36 07 864 ist es bei einer gattungsfremden Anordnung bekannt, daß als Energiequelle für die Lichtenergie entweder diffuses Sonnenlicht oder vergleichbares Licht Verwendung finden kann. Die Art der Gestaltung des Reaktorgefäßes oder die Art der Lichteinleitung in das Reaktorgefäß wird in dieser Druckschrift nicht ausdrücklich angesprochen. Allenfalls entnimmt der Fachmann der Zeichnung dieser Druckschrift den Hinweis im Reaktorgefäß, eine elektrische Beleuchtung zu installieren, da im Reaktorgefäß ein entsprechendes Symbol dargestellt ist.

Bei einer derart kontrollierten Lichteinleitung läßt sich das Betriebsverhalten der gesamten Anordnung der in das Fluid eingeleiteten Abgasmenge exakt anpassen. Die bekannte Anordnung beansprucht sehr viel Platz, der in Form eines eigenen Prozeßraumes bereitgestellt werden muß, der für die Algen günstig klimatisiert werden kann.

Weiterhin ist es aus der Praxis bekannt, ein Reaktorgefäß dadurch zu bilden, daß transparente Rohrleitungen verwendet werden. Die Verlegung der Rohrleitungen erfolgt serpentinenförmig oder meanderförmig und erfordert eine Vielzahl von einzelnen Halteelementen, so daß ein erheblicher Montageaufwand die Kosten für die Einrichtung einer derartigen Anlage ungünstig beeinflußt.

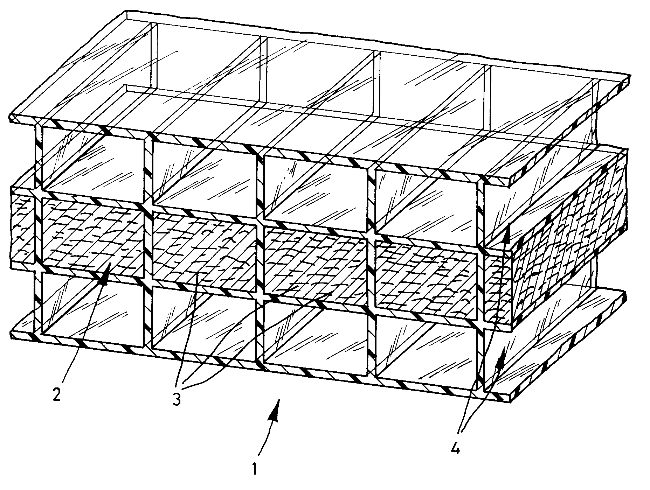

Aus der EP-A-590 477 ist es bei einer gattungsfremden Anordnung bekannt, ein Metalloxid mit photokatalytischen Eigenschaften auf beliebige Baustoff-Oberflächen aufzubringen wie zum Beispiel Wand-oder Dachmaterial odar auch auf Glas. Insbesondere ist es aus den Fig 13 und 14 bekannt, ein Fluid durch ein Flächenelement mit transparenten Oberflächen zu leiten. Das Metalloxid enthält keine lebenden Komponenten, deren Lebensanforderungen berücksichtigt werden müssen.

Der Erfindung liegt die Aufgabe zugrunde, eine gattungsgemäße Anordnung dahingehend zu verbessern, daß sie möglichst platzsparend aufgebaut werden kann, wobei eine schnelle und einfache Verlegbarkeit des Rohrleitungssystemes sowie ein guter Wirkungsgrad gewährleistet ist.

Diese der Erfindung zugrundeliegende Aufgabe wird durch eine Anordnung mit den Merkmalen des Anspruchs 1 gelöst.

Die Erfindung schlägt mit anderen Worten vor, das Flächenelement mit einer thermisch isolierenden Schicht zu versehen, m optimale Arbeitsbedingungen für die Algen sicherzustellen. Auf diese Weise können die Einsatzbedingungen für die Algen vergleichmäßigt werden, so daß der Wirkungsgrad der Anlage nicht in dem Maße schwankt wie die äußeren Witterungsverhältnisse. Auf diese Weise wird der Einsatz der Reaktorgefäße im Freien erleichtert oder je nach regionalen Witterungsbedingungen überhaupt erst ermöglicht.

Die thermische Isolierung vergleichmäßigt die im Fluid herrschende Temperatur, so daß auch bei unterschiedlichen Außentemperaturen die Effizienz der gesamten Anordnung sehr gleichmäßig ist und zuverlässige und ausreichende Arbeitsergebnisse erzielbar sind. Vorteilhaft kann dabei vorgesehen sein, das Flächenelement dreischichtig auszubilden, so daß eine Isolierung einer mittleren fluidführenden Schicht gegenüber beiden äußeren Schichten möglich ist. Auf diese Weise kann eine weitere Vergleichmäßigung der Arbeitsbedingugnen für die Algen geschaffen werden.

Die Ausbildung als Flächenelement ermöglicht die Verwendung als Isolierplatte an Gebäuden, so daß eine platzsparende Anordnung geschaffen wird, die keine zusätzlichen Aufstellflächen für diese Abschnitte des Rohrleitungssystems benötigt.

Vorteilhaft kann das Flächenelement als Stegplatte ausgebildet sein. Diese an sich bekannte Ausbildung von Flächenelementen ermöglicht eine preisgünstige Herstellung und durch die Vielzahl von in einem Flächenelement vorhandenen Stegen wird eine hohe Druckfestigkeit des gesamten Flächenelementes bewirkt, so daß die thermisch isolierenden Schichten durch die Anlegung eines Unterdruckes evakuiert werden können und hierdurch optimale Isolationsergebnisse erzielt werden können.

Vorteilhaft kann für die Stegplatte ein transparenter oder transluzenter Werkstoff Verwendung finden. Auf diese Weise wird das gesamte Flächenelement lichtdurchlässig, so daß es beispielsweise in Betriebshallen anstelle von Fenstern engesetzt werden kann. Diese sind häufig ohnehin nicht transparent, sondern transluzent ausgebildet, so daß durch die Verwendung der vorgeschlagenen Flächenelemente keine verschlechterten Sichtbedingungen innerhalb der Betriebshallen bewirkt werden. Zusätzliche Aufstellplätze für das Rohrleitungssystem entfallen daher, so daß Beschädigungsgefahren für das Rohrleitungssystem, wie sie auf einem Betriebsgrundstück durch Rangierarbeiten od. dgl. bestehen können, ausgeschlossen werden können. Durch eine Ausbildung des Flächenelementes aus ein und demselben Werkstoff ist das Flächenelement preisgünstig herstellbar, beispielsweise als Stegplatte extrudierbar, so daß eine einheitlich transparente oder transluzente Ausgestaltung ein preisgünstigeres Flächenelement ergeben kann als bei einer Ausbildung mit einer lichtsperrenden und einer lichtdurchlässigen Seite des Flächenelementes.

Die Verwendung von Polykarbonat als Werkstoff ermöglicht dabei eine verschleißfeste und sehr leichtgewichtige Konstruktion eines Flächenelementes.

Vorteilhaft kann die Isolationswirkung de thermisch isolierenden Schichten durch ein angelegtes Vakuum bzw. durch einen angelegten Unterdruck erzielt werden. Hierdurch ergibt sich einerseits ein sehr geringer Energieverbrauch, da das einmal aufgebaute Vakuum lediglich gehalten werden muß, so daß die Unterdruckpumpen lediglich intermittierend eingeschaltet werden müssen. Gleichzeitig wird eine hervorragende Isolationswirkung in den evakuierten Schichten bewirkt, so daß eine erhebliche Steigerung der Effizienz bei der Arbeitsweise der Algen erzielt werden kann. Insbesondere kann diese Effizienz durch eine ständige Aufrechterhaltung des Vakuums beibehalten werden, da Leckagen, z. B. durch Diffusion, nicht zu einer schleichenden Verschlechterung der Isolierwirkung führen, sondern durch die angeschlossenen Unterdruckpumpen stets ausgeglichen werden.

Schließlich ermöglicht ein derartig angelegter Unterdruck die schnelle und zuverlässige Erkennung von Leckagen, da aus den fluidführenden Kanälen austretendes Fluid mit Hilfe der Unterdruckpumpen durch die evakuierten Schichten gefördert wird.

Ein Ausführungsbeispiel eines Flächenelementes, wie es in einer erfindungsgemäßen Anordnung vorgesehen sein kann, wird anhand der Zeichnung im folgenden näher erläutert.

Dabei ist mit 1 allgemein ein Flächenelement bezeichnet, welches als Dreischicht-Stegplatte ausgebildet ist. Das Flächenelement 1 weist eine mittlere Schicht 2 auf, die aufgrund der Stege aus einer Vielzahl einzelner Kanäle 3 besteht. In dieser mittleren Schicht 2 werden Algen in einem Fluid durch die Kanäle 3 gepumpt. Da das gesamte Flächenelement 1 aus einem transparenten Werkstoff besteht, beispielsweise aus Polykarbonat, sind bei entsprechender Plazierung des Flächenelementes 1 die Algen dem Tageslicht ausgesetzt und ermöglichen die Umsetzung von im Fluid enthaltenem Kohlendioxid zu Sauerstoff.

Zwei äußere Schichten 4 bestehen ebenfalls aus mehreren Kanälen. Diese äußeren Schichten 4 können evakuiert werden, so daß sie thermisch isolierende Eigenschaften erhalten und die Algen innerhalb der mittleren Schicht 2 gegenüber den Umgebungstemperaturen schützen. Bei entsprechenden Temperaturverhältnissen kann auf die Evakuierung ggf. verzichtet werden, so daß die gesamte Anordnung unter Verwendung des Flächenelementes 1 besonders kostengünstig betrieben werden kann.

Das dargestellte Flächenelement 1 kann zwar separat aufgestellt werden, z. B. in einem für die Sonneneinstrahlung optimalen Winkel. Das Flächenelement 1 kann jedoch auch als bautechnisches Flächenelement eingesetzt werden, z. B. für die Abdeckung von Lichtschächten oder Fenstern, wobei aufgrund der beiden äußeren evakuierbaren Schichten 4 das Flächenelement 1 eine hervorragende Isolierwirkung aufweist und zu einer besonders energiesparenden Bauweise, z. B. in klimatisierten oder beheizten Räumen beitragen kann.

Auch bei einer nicht vollständig lichtdurchlässigen Ausbildung des Flächenelementes 1 kann ein derartiges Flächenelement 1 unmittelbar an einem Gebäude angeordnet werden, so daß die isolierenden Eigenschaften des Flächenelementes 1 eine Isolierung des Baukörpers bewirken und die Energiebilanz zum Betreiben dieses Gebäudes verbessern.

Insbesondere in produzierenden Betrieben steht häufig durch Abgase, Abdämpfe od. dgl. ein Energieüberschuß zur Verfügung, der zum Betreigen von Unterdruckpumpen verwendet werden kann, die die beiden isolierenden Schichten 4 evakuieren können.

Die Isolationswirkung des beschriebenen dreischichtigen Ausführungsbeispieles kann auch dadurch erzielt werden, daß eine ein- oder zweischichtige Platte mit zusätzlichen Isolationsschichten kombiniert wird, beispielsweise durch die Anordnung einer lediglich ein- oder zweischichtigen Platte hinter bereits vorhandenen Fenstern oder durch die separate, nicht miteinander verbundene Anordnung von einschichtigen Flächenelementen, beispielsweise einschichtigen Stegplatten.

## Patentansprüche

1. Anordnung zum Aufbereiten kohlendioxidhaltiger Gase mittels eines algenhaltigen Fluids, mit einem beleuchtbaren Reaktorgefäß, in welchem den Algen Lichtenergie zugeführt wird, wobei das Reaktorgefäß als Flächenelement (1) ausgebildet ist, wobei das Flächenelement (1) zumindest an einer von seinen beiden Oberflächen lichtdurchlässig ausgebildet ist, dadurch gekennzeichnet, daß das Flächenelement (1) zwei- oder mehrschichtig ausgebildet ist, wobei eine fluidführende Schicht (2) vorgesehen ist und eine thermisch isolierende Schicht (4).

2. Anordnung nach Anspruch 1, dadurch gekennzeichnet, daß das Flächenelement (1) dreischichtig ausgebildet ist, wobei das Fluid in einer mittleren Schicht (2) geführt ist und beidseitig von dieser fluidführenden Schicht (2) jeweils eine thermisch isolierende Schicht (4) vorgesehen ist.

3. Anordnung nach Anspruch 1 oder 2, gekennzeichnet durch ein als Stegplatte ausgebildetes Flächenelement (1), wobei mittlere Kanäle (3) zur Aufnahme des Fluids vorgesehen sind sowie äußere Hohlräume beiderseits der mittleren Kanäle (3).

4. Anordnung nach Anspruch 3, dadurch gekennzeichnet, daß die Stegplatte als Dreischicht-Stegplatte ausgebildet ist.

5. Anordnung nach einem der vorhergehenden Ansprüche, dadurch gekennzeichnet, daß das Flächenelement (1) insgesamt aus transparentem oder transluzenten Material besteht.

6. Anordnung nach Anspruch 5, dadurch gekennzeichnet, daß das Flächenelement (1) aus Polykarbonat besteht.

7. Anordnung nach einem der vorhergehenden Ansprüche, dadurch gekennzeichnet, daß zur thermischen Isolierung ungefüllte Schichten (4) in dem Flächenelement (1) vorgesehen sind sowie eine Vakuumpumpe zur Evakuierung dieser ungefüllten Schichten (4).

## Claims

1. System for the treatment of gases containing carbon dioxide by means of a fluid containing algae, comprising a reactor vessel which is adapted to be illuminated, in which light energy is supplied to the algae, the reactor vessel being in the form of a large-area element (1) and being configured to be permeable to light at least on one of its two surfaces, characterised in that the large-area element (1) is configured to be dual or multi-layered, there being provided a fluid-conveying layer (2) and a thermally insulating layer (4).

2. System according to claim 1, characterised in that the large-area element (1) is triple-layered, the fluid being conducted in a middle layer (2) and there being provided a respective thermally insulating layer (4) on either side of this fluid-conveying layer (2).

3. System according to claim 1 or 2, characterised by having a large-area element (1) in the form of a webbed plate, there being provided central channels (3) for receiving the fluid and external cavities on both sides of the central channels (3).

4. System according to claim 1, charactrised in that the webbed plate is in the form of a triple-layered webbed plate.

5. System according to any of the preceding claims, characterised in that the large-area element (1) is on the whole made from a transparent or translucent material.

6. System according to claim 5, characterised in that the large-area element (1) is made from polycarbonate.

7. System according to any of the preceding claims, characterised in that for thermal insulation unfilled layers (4) are provided in the large-area element (1), as is a vacuum pump for evacuating these unfilled layers (4).

## Revendications

1. Dispositif pour traiter des gaz contenant du dioxyde de carbone à l'aide d'un fluide contenant des algues, comportant un réacteur pouvant être éclairé, dans lequel une énergie lumineuse est envoyée sur les algues, le réacteur étant configuré comme un élément bidimensionnel (1), où l'élément bidimensionnel (1) est réalisé de façon à être transparent à la lumière au moins sur l'une de ses deux surfaces, caractérisé en ce que l'élément bidimensionnel (1) est bicouche ou multicouche, auquel cas on prévoit une couche (2) pour le transport du fluide et une couche (4) isolante thermique.

2. Dispositif selon la revendication 1, caractérisé en ce que l'élément bidimensionnel (1) a une configuration tricouche, où le fluide passe dans une couche centrale (2), une couche isolante thermique (4) étant prévue de chaque côté de cette couche (2) destinée au passage du fluide.

3. Dispositif selon la revendication 1 ou 2, caractérisé par un élément bidimensionnel (1), configuré comme une traverse, où des canaux centraux (3) sont prévus pour recevoir le fluide, ainsi que des cavités extérieures des deux côtés des canaux centraux (3).

4. Dispositif selon la revendication 3, caractérisé en ce que la plaque de traverse est configurée comme une plaque de traverse tricouche.

5. Dispositif selon l'une des revendications précédentes, caractérisé en ce que l'élément bidimensionnel (1) est globalement constitué d'un matériau transparent ou translucide.

6. Dispositif selon la revendication 5, caractérisé en ce que l'élément bidimensionnel (1) est constitué de polycarbonate.

7. Dispositif selon l'une des revendications précédentes, caractérisé en ce que, pour assurer l'isolation thermique, des couches non remplies (4) sont prévues dans l'élément bidimensionnel (1), ainsi qu'une pompe à vide destinée à faire le vide dans ces couches non remplies (4).
